# EUROPEAN PATENT APPLICATION

(11) **EP 4 434 518 A1**
(43) Date of publication of application: **25.09.2024**
(21) Application number: 23763759.0
(22) Date of filing: 03.03.2023
(51) Int. Cl.: A61K 31/05, A61K 47/44, A61P 15/00, A61P 15/08, A61K 9/00

(54) **COMPOSITION FOR IMPROVING REPRODUCTIVE ABILITY OF COW**

(30) Priority: 04.03.2022 KR 20220028114; 08.06.2022 KR 20220069430
(71) Applicant: Solomon Co., Ltd., Sangju-si, Gyeongsangbuk-do 37224 (KR)
(72) Inventor: YOON, Minjung, Yongin-si Gyeonggi-do 16856 (KR); KIM, Ho-youn, Gangneung-si Gangwon-do 25451 (KR); KIM, Junyoung, Sangju-si Gyeongsangbuk-do 37224 (KR)
(74) Representative: BCKIP Part mbB
(86) International application number: PCT/KR2023/002976
(87) International publication number: WO 2023/167561

(57) **Abstract**

The present invention relates to a composition for improving cow reproductive ability, capable of not only improving productivity by improving the reproductive ability of cows, but also preventing various problems that may occur by indiscriminate use of hormonal preparations, and alleviating the economic burden of breeders, such as feed costs.

## Description

### [Technical Field]

The present invention relates to a composition for improving cow reproductive ability. The present invention is invented with the support of project title "Development and empirical evaluation of technology for improving cow reproductive ability using hydrogen-specific pheromone" (project No.: 1545026112/ project management institution: Korea Institute of Planning and Evaluation for Technology in Food, Agriculture, and Forestry/ Research period: April 1, 2022 to December 31, 2023) and project title "KIST-Industry Bridge Program" (project No.: 1711173223/ project management institution: Korea Institute of Science and Technology).

### [Background Art]

According to a recent study result, the share of feed cost in the management cost of Hanwoo farms in Korea increased from 31.8% in 2005 to 40.6% in 2018. While the share of feed costs has been maintained high at 40.6 to 48.8% since 2010, the income per head has decreased from 1,962,000 won in 2016 to 1,088,000 won in 2018. In addition, the net profit obtained by deducting production cost from crude profit was 988,000 won in 2016, but decreased significantly to 133,000 won in 2017, and the net loss of 57,000 won occurred in 2018, worsening the management balance. As a result, the price of assorted feed for cattle continued to rise, and it was known that the price soared by about 18%, approaching 457 won/kg in 2021 compared to the normal year (388 won/kg).

On the other hand, farms for Hanwoo and dairy cattle are suffering from reduced productivity due to reproductive disability of cows, such as delayed return of estrus after calving, increased frequency of weak estrus, and irregular estrus period of cows, and the burden of production cost is increasing according to an increase in the amount of feed wasted according to a non-pregnant period of the cows. In 2018, according to study results of evaluation and analysis of the reproductive status for 343 heads of reproductive cows, a total of 162 cows started to return to estrus within 60 days in actual farms, accounting for only 47% of the total number of cow heads. In addition, the frequent occurrence of weak estrus increased the difficulty of determining the proper time for fertilization, and due to the irregular estrus period, a fertilization success rate was only 65% when artificial fertilization was attempted once. The non-pregnant period is extended due to the delayed return of estrus and the low fertilization success rate after calving of cows, and when fertilization fails continuously, the cows are frequently slaughtered without being used as reproductive cows, and thus, such reproductive disability of cows leads to an increase in the economic burden of breeding farms.

In order to solve these problems, in most farms, synthetic GnRH preparations are injected to induce estrus in cows, but due to tolerance problems caused by continuous use thereof, the amount of injection is gradually increasing, and finally, not only there is a concern that consumers' preference for livestock used as food may decrease, but also the possibility of ingesting residual drugs according to meat intake remains to cause a problem. In addition, continuous and indiscriminate injection of hormone preparations may cause stress to livestock, and may act as a risk factor that increases labor of breeders and threatens the safety of workers during injection. In addition, even if estrus is induced using an injection, there are many cases where the proper time for fertilization is missed, and thus, there is a limit to ideally increasing the actual artificial insemination success rate.

According to a report in the United States, it was confirmed that when 60 cows and 1 bull were grazed together, the time to return to estrus after calving and the time to show estrous behavior were accelerated by at least 20 days compared to a group in which only cows were grazed (Landaeta-Hernandez, et al. (2008)). Accordingly, the present invention aims to confirm an effect of improving cow reproductive ability of a specific pheromone contained in the urine of bulls, and to provide a composition for improving cow reproductive ability using the same.

### [Disclosure]

### [Technical Problem]

In order to solve economic problems that may occur due to reproductive disability of cows, such as delay in return to estrus, increased frequency of weak estrus, irregular estrus period of cows, an object of the present invention is to provide a composition capable of improving cow reproductive ability.

### [Technical Solution]

An aspect of the present invention provides a composition for improving cow reproductive ability including *p*-cresol as an active ingredient.

In the present invention, the composition may preferably include at least one wax selected from the group consisting of paraffin wax, beeswax, wood wax, and carnauba wax.

In the present invention, the *p*-cresol may be included in an amount of preferably 0.1 to 5 wt% based on the entire composition. The *p*-cresol may be included in preferably 0.5 to 3 wt%, more preferably 1 wt% based on the entire composition.

Another aspect of the present invention provides a method for preparing a composition for improving cow reproductive ability, including mixing *p*-cresol with at least one wax selected from the group consisting of paraffin wax, beeswax, wood wax and carnauba wax.

In the present invention, the wax may be preferably in a liquid state.

In the present invention, the composition may be mixed with preferably 95 to 99.9 wt% of the wax and 0.1 to 5 wt% of the p-cresol. The composition may be mixed with preferably 97 to 99.5 wt% of the wax and 0.5 to 3 wt% of the *p*-cresol, more preferably 99 wt% of the wax and 1 wt% of the *p*-cresol.

Yet another aspect of the present invention provides a method for improving cow reproductive ability, including applying the composition to cows.

### [Advantageous Effects]

According to the present invention, it is possible to provide a composition for improving cow reproductive ability capable of not only improving productivity by improving the cow reproductive ability, but also preventing various problems that may occur by indiscriminate use of hormonal preparations, and alleviating the economic burden of breeders, such as feed costs.

### [Description of Drawings]

FIG. 1 is a diagram of confirming that a large amount of p-cresol is present only in the urine of bulls in an embodiment of the present invention.
FIG. 2 is a diagram of confirming that a large amount of p-cresol is present only in the urine of bulls in an embodiment of the present invention.
FIG. 3 is a diagram illustrating an example of a composition for improving cow reproductive ability prepared in the present invention according to an embodiment of the present invention.

### [Best Mode]

The present invention relates to a composition for improving cow reproductive ability including *p*-cresol as an active ingredient.

The *p*-cresol is also known as 4-methylphenol and refers to a compound represented by Chemical Formula 1 below:

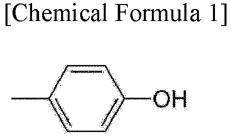

In the present invention, the improving of the cow reproductive ability refers to an effect of shortening a non-pregnant period of the cow according to the shortening of the first return to estrus after calving of the cow.

In the present invention, the return to estrus day means a period from weaning to estrus, that is, the number of days when it takes for the mother cow to come into estrus after the calf is weaned.

Further, the present invention provides a method for preparing a composition for improving cow reproductive ability, including mixing *p*-cresol with at least one wax selected from the group consisting of paraffin wax, beeswax, wood wax and carnauba wax.

In the present invention, the wax may be preferably in a liquid state. It is preferable to prepare the composition by making solid wax completely into a liquid state by heating it in boiling water and mixing the liquid wax with *p*-cresol.

Further, the present invention provides a method for improving cow reproductive ability, including applying a composition for improving cow reproductive ability to cows.

In the present invention, the applying of the composition to the cows is preferably to allow the cow to inhale *p*-cresol diffused from the composition, and for example, means that the composition is mounted on the neck of the cow or mounted in a breeding place (barn, etc.).

### [Modes of the Invention]

Hereinafter, the present invention will be described in more detail through Examples. However, these Examples are more specifically illustrative of the present invention, and the scope of the present invention is not limited to these Examples.

### <Example 1> Confirmation of volatile components contained in bull's urine

In order to solve the problem of reproductive disability in cows due to the absence of bulls, it was confirmed what components were contained in the urine of bulls.

Volatile components contained in each urine were compared through a solid phase microextraction (SPME) test method. Urine was extracted from 16 bulls and 4 steers, respectively. 2 ml of urine for each individual bull was extracted three times. After a septum of a sample container was formed while a fiber was fully inserted, the fiber was exposed to the outside and an analyte was extracted using a headspace method for about 1 hour. After the extraction, when the fiber was inserted again, the fiber was exposed in an inlet of a GC, and the adsorbed analyte was thermally desorbed at a high temperature. Thereafter, the analyte was automatically injected into an analysis column, and then numerical analysis was performed for each material.

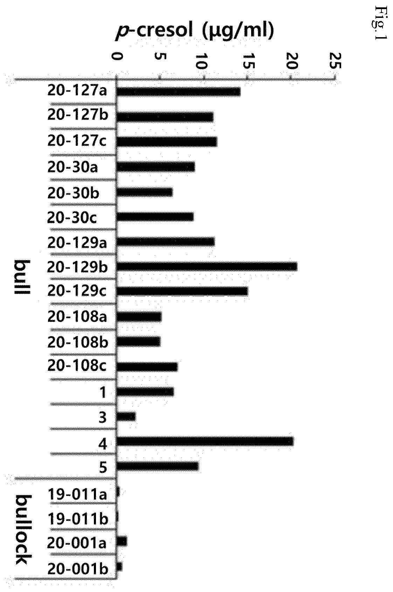

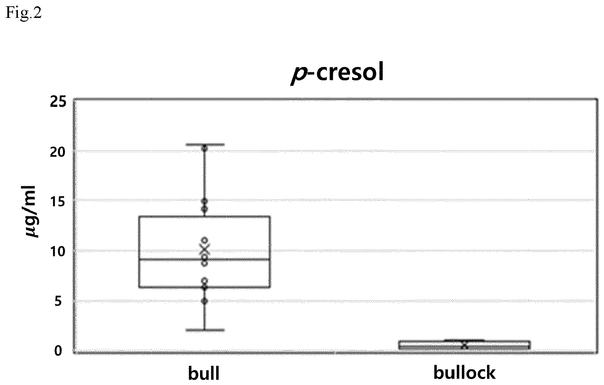

As illustrated in FIGS. 1 and 2, a large amount of *p*-cresol was detected only in the urine of bulls, and was almost not detected in the urine of the steers.

### <Example 2> Preparation of composition for improving cow reproductive ability containing p-cresol

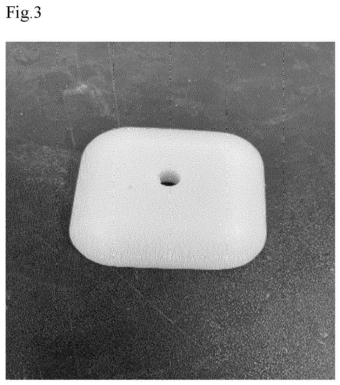

Completely melted paraffin wax and *p*-cresol were mixed in a mixing ratio shown in Table 1 below while stirring. The mixture was poured into a wax tray and completely hardened to prepare a composition for improving cow reproductive ability. FIG. 3 shows a photograph of the appearance of the prepared composition.

**[Table 1]**

| | Paraffin wax | *p*-cresol |
|---|---|---|
| Preparation Example 1 | 99.9 g | 0.1 g |
| Preparation Example 2 | 99 g | 1 g |
| Preparation Example 3 | 95 g | 5 g |

### <Example 3> Confirmation of effect of improving cow reproductive ability

By using the composition prepared in Example 2, the effect of improving the cow reproductive ability was confirmed after being mounted on the leash of cows after calving. The first return to estrus after calving was generally known to be 60 to 90 days, and in the experiment, confirmed to be average 84.20 ± 6.64 in the case of cow used as a control group. The experimental results were shown in Table 2.

**[Table 2]**

| Treatment method | | | Number of heads | First return to estrus day after calving (day) |
|---|---|---|---|---|
| Experimental group | Concentration | Type | | |
| | 5% | Leash mounting type | 3 | 28 + 4 |
| | 1% | | 3 | 46 + 4 |
| Control | | | 5 | 73.86 ± 8.15 |

As shown in Table 2, it was confirmed that *p*-cresol was diffused from the composition of the present invention to improve the cow reproductive ability. In particular, in the case of treatment with a 5% *p*-cresol pheromone block, the estrus returned at day 28 after calving, which was about 17 days earlier than the earliest normal return time of estrus (approximately 45 days after calving). However, in the case of using the 5% *p*-cresol pheromone block, the side effects of shortening the uterine recovery period of the cow are expected, so that it is preferable to use 1% or less of *p*-cresol pheromone block.

## Claims

1. A composition for improving cow reproductive ability comprising *p*-cresol as an active ingredient,
wherein the improving of the cow reproductive ability is to shorten a first return to estrous day after calving.

2. The composition for improving the cow reproductive ability of claim 1, wherein the composition includes at least one wax selected from the group consisting of paraffin wax, beeswax wax, wood wax and carnauba wax.

3. The composition for improving the cow reproductive ability of claim 1, wherein the *p-*cresol is included in an amount of 0.1 to 5 wt% based on the entire composition.

4. A method for preparing a composition for improving cow reproductive ability, comprising mixing *p*-cresol with at least one wax selected from the group consisting of paraffin wax, beeswax, wood wax and carnauba wax,
wherein the improving of the cow reproductive ability is to shorten a first return to estrous day after calving.

5. The method for preparing the composition for improving cow reproductive ability of claim 4, wherein the wax is in a liquid state.

6. The method for preparing the composition for improving cow reproductive ability of claim 4, wherein the composition is mixed with 95 to 99.9 wt% of the wax and 0.1 to 5 wt% of the *p-*cresol.

7. A method for improving cow reproductive ability, comprising mounting the composition of claim 1 to a body part of the cow or to a breeding place,
wherein the improving of the cow reproductive ability is to shorten a first return to estrous day after calving.
